(19) Europäisches Patentamt
European Patent Office
Office européen des brevets

(11) EP 4 148 055 A1

(12) **EUROPEAN PATENT APPLICATION**
published in accordance with Art. 153(4) EPC

(43) Date of publication:
**15.03.2023 Bulletin 2023/11**

(21) Application number: **21800713.6**

(22) Date of filing: **07.05.2021**

(51) International Patent Classification (IPC):
***C07D 487/04*** *(2006.01)* ***A61K 31/519*** *(2006.01)*
***A61P 35/00*** *(2006.01)*

(52) Cooperative Patent Classification (CPC):
**A61K 31/519; A61K 31/5395; A61P 25/00; A61P 25/16; A61P 25/28; A61P 29/00; A61P 35/00; A61P 37/02; C07D 487/04; C07D 498/22**

(86) International application number:
**PCT/CN2021/092225**

(87) International publication number:
**WO 2021/223748 (11.11.2021 Gazette 2021/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.05.2020 CN 202010382192**

(71) Applicants:
- **Shandong Xuanzhu Pharma Co., Ltd.**
**National High-Tech Development Zone Jinan Shandong 250101 (CN)**
- **Xuanzhu Biopharmaceutical Co., Ltd.**
**Shijiazhuang, Hebei 050000 (CN)**

(72) Inventors:
- **WU, Zhanying**
**Jinan, Shandong 250101 (CN)**
- **ZHU, Peng**
**Jinan, Shandong 250101 (CN)**
- **ZHANG, Chongkun**
**Jinan, Shandong 250101 (CN)**

(74) Representative: **Finetti, Claudia**
**Bugnion S.p.A.**
**Viale Lancetti, 17**
**20158 Milano (IT)**

(54) **CRYSTAL FORM OF MACROCYCLIC TYROSINE KINASE INHIBITOR AND PREPARATION METHOD THEREFOR**

(57) The present disclosure relates to a crystal form of a macrocyclic tyrosine kinase inhibitor and a preparation method therefor, specifically relates to a crystal form of a compound represented by Formula (I) and a preparation method therefor, a pharmaceutical formulation and pharmaceutical composition containing the crystal form, and an application thereof in preparing drugs for treating and/or preventing pain, inflammation, cancer, neurodegenerative diseases, and autoimmune diseases mediated by one or more tyrosine kinase receptors in a tropomyosin receptor kinase (TRK), an anaplastic lymphoma kinase (ALK) and/or a c-ros oncogene 1 receptor kinase (ROS1).

EP 4 148 055 A1

## Description

### Cross-Reference to Related Application

The present application is based upon and claims priority to CN application No. 202010382192.2, filed on May 8, 2020, the disclosure of which is hereby incorporated by reference in its entirety.

### Technical Field

The present disclosure relates to a crystal form of a macrocyclic tyrosine kinase inhibitor, a preparation method therefor and a pharmaceutical composition or formulation thereof. Herein the macrocyclic tyrosine kinase is one or more of a tropomyosin receptor kinase (TRK), an anaplastic lymphoma kinase (ALK) and/or a c-ros oncogene 1 receptor kinase (ROS1). The present disclosure further relates to an application thereof in preparing drugs for treating and/or preventing pain, inflammation, cancer, neurodegenerative diseases, and autoimmune diseases mediated by one or more tyrosine kinase receptors in TRK, ALK and/or ROS1.

### Background

A molecular targeted therapy is a major breakthrough in cancer treatment in recent years. Compared with traditional treatment methods such as asurgery, a radiotherapy and a chemotherapy, the molecular targeted therapy opens up a new world for the cancer treatment with its high specificity and relatively low side effects, and gradually becomes a standard treatment scheme for patients with advanced cancer. As a major field of the targeted therapy, a protein kinase is a key regulator of cell growth, proliferation and survival, and its genetic and epigenetic alterations may lead to the occurrence of cancer.

ALK belongs to an insulin receptor superfamily of a receptor tyrosine kinase, and plays an important role in brain development and specific neurons. At present, ALK mutations have already been found in avariety of cancers, including anaplastic large cell lymphoma (ALCL), non-small cell lung cancer, inflammatory myofibroblastic tumor, colorectal cancer, breast cancer and several other cancers.

TRK is a high-affinity receptor for a neurotrophic protein (NT). Members of a TRK family are highly expressed in cells of neural origin. Because TRK plays an important role in pain perception and tumor cell growth and survival signal transduction, inhibitors of a TRK receptor kinase may provide benefits as pain and cancer therapeutic agents.

ROS1 is also a tyrosine kinase receptor that attracts much attention at present. It is already reported that ROS1 undergoes gene rearrangement to produce a constitutive active fusion protein in many human cancers, and the cancers include glioblastoma, the non-small cell lung cancer, the colorectal cancer, the breast cancer and the like.

The above three tyrosine kinases have stronger homology. ROS1 gene and ALK gene have 49% of the homology in a tyrosine kinase region sequence, while the homology of the two is up to 77% in an adenosine triphosphatase (ATP) binding site of a kinase catalytic region, the kinase region sequence of TRK A/B/C has more than 80% of the homology, and TRK A gene, ROS1 gene and ALK gene have about 40% of the homology in the tyrosine kinase region sequence. A marketed ALK inhibitor (Crisotinib) has both ROS1 and TRK inhibitory activities, and a TRK inhibitor (Entrectinib) also has ALK and ROS1 inhibitory activities.

At present, the ALK/ROS1 inhibitor that is already marketed and the NTRK inhibitor that is declared to be marketed in 2017 have all experienced drug resistance in the course of long-term drug use. Therefore, it is of great clinical value and significance to develop an anti-tumor drug with strong efficacy, low toxicity, selective inhibitory activity and ability to solve the problem of the drug resistance. At the same time, the research of crystal forms plays an important role in drug research and development. The different crystal forms of the same drug have significant differences in aspects of solubility, stability, bioavailability and the like. In order to better control the quality of drugs and meet the requirements of formulations, production, transportation, storage and the like, crystal forms of compounds are researched in order to find a crystal form with good properties.

### Summary

The present application provides a tyrosine kinase inhibitor with a chemical name ($2^2$*R*, 6*R*) -$3^5$-fluoro-6-methyl-$1^3$*H*-4-oxa-7-aza-1(5,3)-imidazo[4,5-b]pyridina-3(3,2)-pyridina-2(1,2)-pyrrolidina cyclooctaphan-8-one (compound represented by Formula (I) ). The compound has a better inhibitory activity against one or more kinases of TRK, ALK and ROS1, and has a higher exposure and bioavailability in vivo. It is improved in aspects of clinical efficacies or indications, safety and the like.

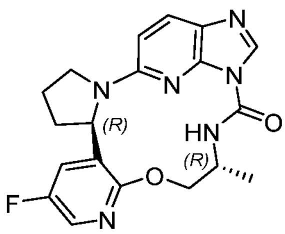

(I)

In another aspect, the present disclosure further relates to a crystal form II of the compound ($2^2R$,$6R$)-$3^5$-fluoro-6-methyl-$1^3H$-4-oxa-7-aza-1(5,3)-imidazo[4,5-b]pyridina-3(3,2)-pyridina-2(1,2)-p yrrolidinacyclooctaphan-8-one represented by Formula (I).

The present disclosure further relates to a preparation method for the crystal form II of the compound represented by Formula (I), a pharmaceutical formulation containing the crystal form II, a pharmaceutical composition containing the crystal form II, and an application of the crystal form II of the compound represented by Formula (I), the pharmaceutical formulation containing the crystal form II, and the pharmaceutical composition containing the crystal form II in preparing drugs for treating and/or preventing pain, inflammation, cancer, neurodegenerative diseases, and autoimmune diseases mediated by one or more tyrosine kinase receptors in TRK, ALK and/or ROS1.

The present disclosure provides the crystal form II of the compound represented by Formula (I) having characteristic peaks at angles (2θ) 10.07±0.2°, 11.01±0.2°, 12.54±0.2°, 14.13±0.2°, 15.96±0.2°, 16.70±0.2° and 21.60±0.2° in an X-ray powder diffraction pattern using Cu-Kα radiation.

In some implementation schemes, the crystal form II has characteristic peaks at angles (2θ) 8.86±0.2°, 13.43±0.2°, 15.06±0.2°, 18.66±0.2°, 19.10±0.2°, 19.71±0.2°, 20.42±0.2° and 22.95±0.2° in an X-ray powder diffraction pattern using Cu-Kα radiation, in addition to the above characteristic peaks.

In some implementation schemes, the crystal form II has characteristic peaks at angles (2θ) 8.86±0.2°, 10.07±0.2°, 11.01±0.2°, 12.54±0.2°, 13.43±0.2°, 14.13±0.2°, 15.06±0.2°, 15.96±0.2°, 16.70±0.2°, 18.66±0.2°, 19.10±0.2°, 19.71±0.2°, 20.42±0.2°, 21.60±0.2°, and 22.95±0.2° in an X-ray powder diffraction pattern using Cu-Kα radiation.

In some implementation schemes, the crystal form II has the X-ray powder diffraction pattern substantially the same as Fig. 1.

In some implementation schemes, a differential scanning calorimetry (DSC) thermogram shows that the crystal form II has an endothermic transition peak at about 155°C-170°C.

In some implementation schemes, the DSC thermogram shows that the crystal form II has an endothermic transition peak at 160°C -165°C.

In some implementation schemes, the DSC thermogram shows that a maximum endothermic transition temperature (phase transition temperature), namely the temperature at the peak of an endothermic peak, of the crystal form II is 162.25±3°C.

In some implementation schemes, the crystal form II has a DSC thermogram substantially the same as Fig. 2.

In some implementation schemes, a thermogravimetry (TGA) diagram shows that the crystal form II has no apparent weight loss at 0°C-250°C.

In some implementation schemes, the crystal form II has a TGA diagram substantially the same as Fig. 3.

In another aspect, the present disclosure further provides a preparation method for the crystal form II of the compound represented by Formula (I), and steps thereof include: mixing the compound represented by Formula (I) with an organic solvent, heating until the compound is completely dissolved, dropping a poor solvent, separating out a solid, filtering and drying, to obtain the crystal form II.

In some implementation schemes, the preparation method for the crystal form II of the compound represented by Formula (I) further includes an operation of cooling.

In some implementation schemes, the cooling operation is performed before the operation of dropping the poor solvent.

In some implementation schemes, the cooling operation is performed after the operation of dropping the poor solvent.

In some implementation schemes, the cooling operation is performed simultaneously with the operation of dropping the poor solvent.

In some implementation schemes, in the preparation method for the crystal form II of the compound represented by Formula (I) provided by the present disclosure, steps thereof include : mixing the compound represented by Formula (I) with an organic solvent, stirring and heating to 30-80°C, until the compound is completely dissolved, cooling to 0-30°C, dropping a poor solvent, stirring at a constant temperature, separating out a solid, filtering and drying, to obtain the crystal form II.

In some implementation schemes, in the preparation method for the crystal form II of the compound represented by Formula (I) provided by the present disclosure, steps thereof include: mixing the compound represented by Formula (I) with an organic solvent, stirring and heating to 30-80°C, until the compound is completely dissolved, dropping a poor solvent, cooling to 0-30°C, stirring at a constant temperature, separating out a solid, filtering and drying, to obtain the crystal form II.

In some implementation schemes, in the preparation method for the crystal form II of the compound represented by Formula (I), it includes an operation of heating to 40-70°C, such as heating to 40-45°C, such as heating to 45-50°C, such as heating to 50-55°C, such as heating to 55-60°C, such as heating to 60-65°C, such as heating to 65-70°C, and such as heating until the sample is dissolved and becomes clear.

In some implementation schemes, in the preparation method for the crystal form II of the compound represented by Formula (I), it includes an operation of cooling to 0-30°C, such as cooling to 0-10°C, such as cooling to 10-15°C, such as cooling to 10-20°C, such as cooling to 15-20°C, and such as cooling to 20-30°C. During the cooling process, the cooling may be performed optionally at different temperatures for many times, and cooling modes thereof include, but are not limited to, natural cooling, ice bath cooling, oil bath cooling, and cooling by a refrigeration device and the like. In the present disclosure, the natural cooling, the oil bath cooling, and the cooling by the refrigeration device are preferred.

In the present disclosure, the "temperature while dropping the poor solvent" refers to the temperature of a reaction system while dropping the poor solvent, rather than the solvent temperature of the poor solvent.

In some implementation schemes, the poor solvent is dropped after cooling until a crystal is separated out. In some implementation schemes, the poor solvent is dropped after cooling but a crystal is not separated out. In some implementation schemes, the cooling operation is completed prior to the operation of dropping the poor solvent. In some implementation schemes, the cooling operation is completed later than the operation of dropping the poor solvent. In some implementation schemes, the cooling operation and the operation of dropping the poor solvent are completed simultaneously.

In some implementation schemes, in the preparation method for the crystal form II of the compound represented by Formula (I), the temperature while dropping the poor solvent is selected from 0-30°C, such as 0-10°C, such as 10-15°C, such as 15-20°C, and such as 20-30°C.

In some implementation schemes, in the preparation method for the crystal form II of the compound represented by Formula (I), the cooling operation is performed before the operation of dropping the poor solvent; and , the temperature of the reaction system while dropping the poor solvent is, such as 0-30°C, such as 0-10°C, such as 10-15°C, such as 10-20°C, such as 15-20°C, and such as 20-30°C.

In some implementation schemes, in the preparation method for the crystal form II of the compound represented by Formula (I), the temperature while dropping the poor solvent is selected from 10-70°C, such as 20-60°C, such as 55-65°C, such as 45-55°C, such as 35-45°C, and such as 20-30 °C.

In some implementation schemes, the cooling is started after the operation of dropping the poor solvent is completed. In some implementation schemes, the cooling is started after the operation of dropping the poor solvent is started but before it is completed. In some implementation schemes, the cooling operation is completed prior to the operation of dropping the poor solvent. In some implementation schemes, the cooling operation is completed later than the operation of dropping the poor solvent. In some implementation schemes, the cooling operation and the operation of dropping the poor solvent are completed simultaneously.

In some implementation schemes, in the preparation method for the crystal form II of the compound represented by Formula (I), the cooling operation is performed after the operation of dropping the poor solvent; and the temperature while dropping the poor solvent is selected from 10-70°C, such as 20-60°C, such as 55-65°C, such as 45-55°C, such as 35-45°C, and such as 20-30 °C.

In some implementation schemes, in the preparation method for the crystal form II of the compound represented by Formula (I), the organic solvent is selected from one of a ketone solvent and an ester solvent, or a mixed solvent formed by combining two or more solvents in a specific proportion. In some implementation schemes, the ketone solvent is selected from fatty ketone and cycloketone solvents. In some implementation schemes, the fatty ketone solvent is preferably a methyl ethyl ketone, a methyl isopropylacetone, an acetone, a methyl butanone or a methyl isobutyl ketone, more preferably the acetone. And the cycloketone solvent is preferably a cycloacetone, a cyclohexanone, an isophorone or an N-methylpyrrolidone, more preferably the N-methylpyrrolidone. In some implementation schemes, the ester solvent is selected from fatty ester and aromatic ester solvents. In some implementation schemes, the fatty ester solvent is preferably a methyl formate, an ethyl formate, a propyl formate, a methyl acetate, an ethyl acetate, a propyl acetate, an isopropyl acetate, a butyl acetate, an isobutyl acetate, a methyl propionate, an ethyl propionate, a propyl propionate or an isopropyl propionate, further preferably the ethyl formate, the methyl acetate, the ethyl acetate or the propyl acetate, and more preferably the ethyl acetate, and the aromatic ester solvent is such as a dimethyl phthalate.

The ketone solvent and the ester solvent mentioned above are not limited to specific examples listed, and all solvents belonging to the above classification that may be used to prepare the crystal form II of the compound represented

by Formula (I) fall within a scope of protection of the present disclosure.

The "mixed solvent" refers to a solvent formed by mixing the same type or different types of solvents in the above the organic solvents in a certain proportion. The mixed solvent formed by the same type of the solvents includes but is not limited to the following specific examples: methyl formate/ethyl acetate, methyl acetate/ethyl acetate, isobutyl acetate/ethyl acetate or methyl ethyl ketone/acetone and the like. The mixed solvent formed by the different types of the solvents includes but is not limited to esters/ketones.

In some implementation schemes, in the preparation method for the crystal form II of the compound represented by Formula (I), the organic solvent is selected from one or two of the acetone and the ethyl acetate.

In some implementation schemes, in the preparation method for the crystal form II of the compound represented by Formula (I), the poor solvent is selected from water and n-heptane.

In some implementation schemes, in the preparation method for the crystal form II of the compound represented by Formula (I), the drying mode includes but is not limited to natural drying at a room temperature, infrared lamp drying, oven drying and dryer drying, preferably drying under a vacuum condition; the preferred drying temperature is 30°C-100°C, preferably 30°C-80°C, preferably 35°C-70°C, preferably 40°C-65°C, and preferably 45°C-55°C; and during the drying process, the drying may be performed optionally at different temperatures for many times.

In another aspect, the present disclosure further provides a pharmaceutical formulation or a pharmaceutical composition, and it contains the previously described crystal form II of the compound represented by Formula (I), and one or more pharmaceutically acceptable carriers and/or excipients.

The crystal form II of the compound represented by Formula (I) of the present disclosure may be administered alone, or combined with one or more drugs to treat and/or prevent diseases and related diseases mediated by one or more tyrosine kinases in TRK, ALK and/or ROS1.

Therefore, in some implementation schemes, the pharmaceutical composition further contains one or more second therapeutic active agents. In some implementation schemes, the second therapeutic active agent is selected from a drug for treating pain, a drug for treating cancer, a drug for treating inflammation, a drug for treating neurodegenerative diseases, and a drug for treating autoimmune diseases.

In some implementation schemes, the drug for treating the pain includes but is not limited to a Nav1.7 channel regulator, an opioid analgesic, a non-steroidal anti-inflammatory drug, a sedative, a selective/non-selective cyclooxygenase inhibitor, an antiepileptic drug, an antidepressant, a local anesthetic, a 5-HT receptor blocker, a 5-HT receptor agonist, an ergot alkaloid, a β-receptor blocker, an M receptor blocker, a nitrate, a vitamin K and the like.

In some implementation schemes, the drug for treating the cancer includes but is not limited to a mitotic inhibitor, an alkylating agent, an antimetabolite, an antisense deoxyribonucleic acid (DNA) or ribonucleic acid (RNA), an anti-tumor antibiotic, a growth factor inhibitor, a signal transduction inhibitor, a cell cycle inhibitor, an enzyme inhibitor, a retinoid receptor modulator, a proteasome inhibitor, a topoisomerase inhibitor, a biological response modifier, a hormone drug, an angiogenesis inhibitor, a cell growth inhibitor, a targeting antibody, an HMG-CoA reductase inhibitor, and an isoprene protein transferase inhibitor and the like.

In some implementation schemes, the drug for treating the inflammation includes but is not limited to a steroidal anti-inflammatory drug and a non-steroidal anti-inflammatory drug.

In some implementation schemes, the drug for treating the neurodegenerative diseases includes but is not limited to: a dopaminergic drug, a dopamine metabolism-affecting drug, a drug affecting dopamine metabolism, an N-methyl-D-aspartate (NMDA) receptor antagonist, an adenosine A2A receptor inhibitor, a DA releasing and reuptake-affecting drug, a central anticholinergic drug, a cholinesterase inhibitor, a 5-HT agonist, an $\alpha 2$ adrenergic receptor antagonist, an antidepressant, a choline receptor agonist, a $\beta/\gamma$ secretase inhibitor, an H3 receptor antagonist or an antioxidant drug and the like.

In some implementation schemes, the drug for treating the autoimmune diseases includes but is not limited to: an antirheumatic drug for improving conditions, a non-steroidal anti-inflammatory drug, a glucocorticoid drug, a tumor necrosis factor (TNF) antagonist, a cyclophosphamide, a mycophenolate mofetil, a cyclosporin and the like.

In some implementation schemes, each component to be combined (for example, the crystal form II of the compound represented by Formula (I) of the present disclosure and the second therapeutic active agent) may be administered simultaneously or sequentially separately. For example, the second therapeutic active agent may be administered before, at the same time with, or after the administration of the crystal form II of the compound represented by Formula (I) of the present disclosure. In addition, each component to be combined may also be administered in the form of the same formulation or in the form of separate different formulations.

In some implementation schemes, the pharmaceutical composition may be prepared into any clinically or pharmaceutically acceptable dosage forms, such as a tablet, a capsule, a pill, a granule, a solution, a suspension, a syrup, an injection (including injection solution, sterile powder for injection and concentrated solution for injection), a suppository, an inhalation or a spray and the like.

In some implementation schemes of the present disclosure, the above pharmaceutical formulation or pharmaceutical compositions may be applied to a patient or a subject in need of such treatment by oral, parenteral, rectal or

pulmonary administration and other modes. While used for the oral administration, the pharmaceutical composition may be prepared into an oral formulation. For example, it may be prepared into a conventional oral solid formulation, such as the tablet, the capsule, the pill or the granule. It may also be prepared into an oral liquid formulation, such as the oral solution, the oral suspension or syrup. While prepared into the oral formulation, an appropriate filler, an adhesive, a disintegrant or a lubricant and the like may be added. While used for the parenteral administration, the above drug formulation may also be prepared into the injection, including the injection solution, the sterile powder for injection and the concentrated solution for injection. While prepared into the injection, it may be produced by a conventional method in the existing pharmaceutical field, and while prepared into the injection, an additional agent may not be added, or the appropriate additional agent may also be added according to the nature of the drug. While used for the rectal administration, the pharmaceutical composition may be prepared into the suppository and the like. While used for the pulmonary administration, the pharmaceutical composition may be prepared into the inhalation or the spray and the like.

The pharmaceutically acceptable carriers and/or excipients available in the pharmaceutical composition or pharmaceutical formulation of the present disclosure may be any conventional pharmaceutically acceptable carriers and/or excipients in the field of the pharmaceutical formulations, and the selection of specific carriers and/or excipients may depend on the mode of administration for treating a specific patient or the type and state of a disease. Preparation methods for the suitable pharmaceutical compositions or pharmaceutical formulations for the specific administration modes are completely within the knowledge of those skilled in the pharmaceutical field.

In another aspect, the present disclosure further relates to a use of the previously described crystal form II of the compound represented by Formula (I) in preparing a drug for treating and/or preventing diseases and related diseases mediated by a tyrosine kinase, and the drug may be combined with one or more other drugs to prevent or treat the diseases and related diseases mediated by the tyrosine kinase. The diseases and related diseases mediated by the tyrosine kinases are preferably diseases and related diseases mediated by one or more tyrosine kinases in TRK, ALK and/or ROS1. The diseases and related diseases mediated by one or more tyrosine kinases in the TRK, ALK and/or ROS1 include but are not limited to pain, cancer, inflammation, neurodegenerative diseases, autoimmune diseases, infectious diseases and the like.

In some implementation schemes, the pain may be pain from any sources or causes, including but not limited to one or more of inflammatory pain, visceral pain, cancer-induced pain, chemotherapy pain, trauma pain, surgery and postoperative pain, labor pain, acute pain, chronic pain, intractable pain, somatic pain, nociceptive pain, neural pain, blood-borne pain, immune-borne pain, endocrine-borne pain, pain caused by metabolic disorder, cardiogenic pain, headache, phantom limb pain and toothache.

In some implementation schemes, the cancer includes but is not limited to one or more of lung cancer, colon cancer, prostate cancer, breast cancer, liver cancer, lymphatic cancer, thyroid cancer, multiple myeloma, soft tissue sarcoma, ovarian cancer, cervical cancer, fallopian tube carcinoma, renal cell carcinoma, gastric cancer, gastrointestinal stromal tumor, bone cancer, basal cell carcinoma, peritoneal cancer, dermatofibroma, pancreatic cancer, esophageal cancer, glioblastoma, head and neck cancer, inflammatory myofibroblast tumor and anaplastic large cell lymphoma, and the lung cancer includes a small cell lung cancer and a non-small cell lung cancer.

In some implementation schemes, the inflammation includes but is not limited to atherosclerosis, allergy, and inflammation caused by infection or injury.

In some implementation schemes, the neurodegenerative diseases include but are not limited to one or more of an Alzheimer's disease, a Parkinson's disease, an amyotrophic lateral sclerosis and a Huntington's disease.

In some implementation schemes, the autoimmune diseases include but are not limited to one or more of a rheumatoid arthritis, a Sjogren's syndrome, a type-I diabetes, and a lupus erythematosus.

In some implementation schemes, the infectious disease is such as a trypanosomiasis.

In another aspect, the present disclosure further provides a method for treating and/or preventing diseases and related diseases mediated by one or more tyrosine kinases in TRK, ALK and/or ROS1, and the method includes administering an effective amount of the previously described crystal form II of the compound represented by Formula (I), and the previously described formulation or pharmaceutical composition to a subject in need; and the diseases and related diseases mediated by one or more tyrosine kinases in TRK, ALK and/or ROS1 are defined above.

Unless otherwise specified in the present application, scientific and technical terms used herein have the meanings commonly understood by those skilled in the art. Meanwhile, in order to better understand the present disclosure, definitions and explanations of some terms are provided below.

In the present application, the position of the absorption peak in the X-ray powder diffraction pattern of each crystal form may be within the range of $\pm 0.2°$ of the specific numerical value of the above present disclosure, for example, within the range of $\pm 0.1°$, and the endothermic transition temperature measured by DSC may be within the range of $\pm 3.0°C$ (for example, $\pm 1.0°C$ or $\pm 2.0°C$) of the above specific numerical value.

It should be understood that slightly different X-ray powder diffraction (XRPD or XRD for short) patterns and peak values may be given with different types of devices or different test conditions. The patterns and peak values of the different crystal forms and the relative strength of each diffraction peak are affected by the purity of the compound,

the sample pretreatment, the scanning speed, the particle diameter and calibration and maintenance of the test device. The numerical value provided may not be regarded as an absolute value.

It should be understood that slightly different endothermic transition temperature readings may be given with different types of the devices or different test conditions. This numerical value may be affected by the purity of the compound, the sample weight, the heating rate, the particle diameter, and the calibration and maintenance of the test device. The numerical value provided may not be regarded as an absolute value.

The present disclosure further uses thermogravimetric analysis (TGA) to analyze a relationship between the degree of crystal form decomposition, sublimation, or evaporation (weight loss) and the temperature. TGA refers to a method of heating at a certain rate to determine the weight of a test sample changed with the temperature. It should be understood that the same type of the crystal form is affected by the sample purity, the particle diameter, the different types of the devices, the different test methods and the like, and the numerical values obtained have certain errors. The temperature at which the crystal form decomposes, sublimates or evaporates may be within the range of ±3.0°C of the specific numerical value disclosed above, for example, within the range of ±2.0°C.

As used herein, terms "room temperature" and "normal temperature" refer to the indoor environment temperature, usually 20-30°C, such as 20-25°C.

As used herein, a term "subject" refers to a healthy individual, an individual suffering from or suspected of suffering from a disease, and it may be a human or a non-human mammal, preferably the human. It usually includes a healthy volunteer, a patient, a test object and a treatment object and the like.

As used herein, a term "effective amount" refers to an amount sufficient to achieve, or at least partially achieve, the desired effect. For example, the effective amount of the treatment refers to an amount that is sufficient to cure or at least partially prevent a disease and its complications of a patient who already suffers from the disease; the effective amount of the prevention refers to an amount sufficient to prevent, stop, or delay the occurrence of the disease. The determination of such effective amount is completely within the competence of those skilled in the art. For example, the effective amount for the therapeutic use may depend on the severity of the disease to be treated, the overall state of the own immune system of the patient, the general situation of the patient, such as age, weight and gender, the mode of drug administration, and other treatments administered simultaneously and the like.

The amount of the drug given to the subject depends on the type and severity of the disease or condition and the characteristics of the subject, such as general health status, age, gender, weight and tolerance to the drug, and also depends on the type of the preparation and the mode of drug administration, as well as the administration cycle or time interval and other factors. Those skilled in the art may determine an appropriate dose according to these factors and other factors.

The main advantages of the crystal form of the compound represented by Formula (I) of the present disclosure, especially the crystal form II, include one or more of the followings:

(1) the preparation method is simple, and suitable for industrial production;

(2) it has good properties, and is convenient for production, detection, drug preparation, transportation and storage;

(3) the purity is high, the residual solvent is less, the stability is good, and the quality is easily controlled;

(4) it has good inhibitory activity against one or more tyrosine kinases in TRK, ALK and/or ROS1, and has good exposure and/or bioavailability in vivo; and

(5) it has good in vivo and in vitro efficacy, and may be used to treat and/or prevent the diseases and related diseases mediated by one or more tyrosine kinases in TRK, ALK and/or ROS1.

## Brief Description of the Drawings

Drawings described herein are used to provide further understanding of the present disclosure, and constitute a part of the present application. Schematic embodiments of the present disclosure and descriptions thereof are used to explain the present disclosure, and do not constitute improper limitation to the present disclosure. In the drawing:

Fig. 1 is an X-ray powder diffraction pattern of a crystal form II of a compound represented by Formula (I), herein the ordinate represents the diffraction intensity, and the abscissa represents the diffraction angle (2θ).

Fig. 2 is a DSC analysis diagram of the crystal form II of the compound represented by Formula (I), herein the ordinate represents the heat flow (W/g), and the abscissa represents the temperature T (°C).

Fig. 3 is a TGA analysis diagram of the crystal form II of the compound represented by Formula (I), herein the ordinate represents the weight (%), and the abscissa represents the temperature T (°C).

## Detailed Description of the Embodiments

Technical solutions in embodiments of the present disclosure are clearly and completely described below in combination with the drawings in the embodiments of the present disclosure. Apparently, the described embodiments are only a part of the embodiments of the present disclosure, not all of the embodiments. The following description of at least one exemplary embodiment is only illustrative in fact, and in no way serves as any limitation to the present disclosure and its application or use. Based on the embodiments of the present disclosure, all other embodiments obtained by those of ordinary skill in the art without creative work belong to a scope of protection of the present disclosure.

Embodiment 1: Preparation of ($2^2R$,$6R$)-$3^5$-fluoro-6-methyl-$1^3H$-4-oxa-7-aza-1(5,3)-imidazo[4,5-b]pyridina-3(3,2)-pyridina-2(1,2)-p yrrolidinacyclooctaphan-8-one (compound represented by Formula (I) )

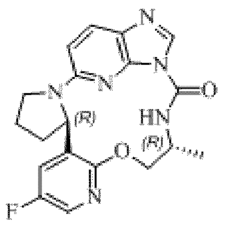

1. Preparation of (R)-6-(2-(5-fluoro-2-methoxypyridin-3-yl)pyrrolidin-1-yl)-3-nitropyridinyl-2-amine

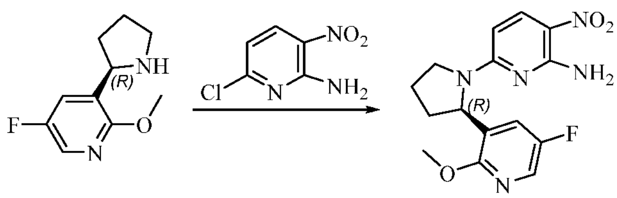

(*R*)-5-fluoro-2-methoxy-3-(pyrrolidin-2-yl)pyridine (1.0 g, 5.1 mmol) and 6-chloro-3-nitropyridinyl-2-amine (886 mg, 5.1 mmol) was added to acetonitrile (30 mL), diisopropylethylamine (1.98 g, 15.3 mmol) was added to obtain a mixture. And the mixture was heated to 70°C, and reacted for 16 hours. Reaction solution was spin-dried, water (30 mL) was added, and filtered. A solid was washed with ethyl acetate (30 mL), and dried in vacuum, to obtain the target product (1.65 g, yield: 97.0%).

LC-MS (M/e) : 334.1 (M+H+)

2. Preparation of (*R*)-6-(2-(5-fluoro-2-methoxypyridin-3-yl)pyrrolidin-1-yl)pyridinyl-2,3-diamine

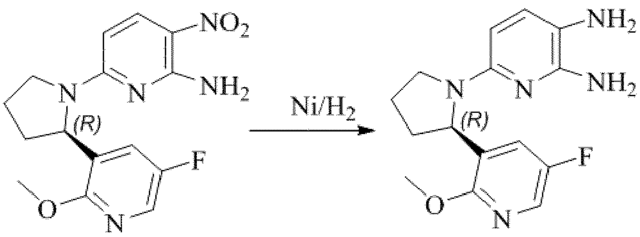

(*R*)-6-(2-(5-fluoro-2-methoxypyridin-3-yl)pyrrolidin-1-yl)-3-nitropyridinyl-2-amine(1.65 g, 4.94 mmol) was dissolved in methanol (20 mL), Raney nickel (1 g) was added to obtain a mixture. And the mixture was reacted for 16 hours under hydrogen environment at 20°C. Reaction solution was filtered and spin-dried, and the residue was directly used in the next step.

LC-MS (M/e) : 304.1 (M+H+)

3. Preparation of (*R*)-5-(2-(5-fluoro-2-methoxypyridin-3-yl)pyrrolidin-1-yl)-3H-imidazo[4,5-*b*] pyridine

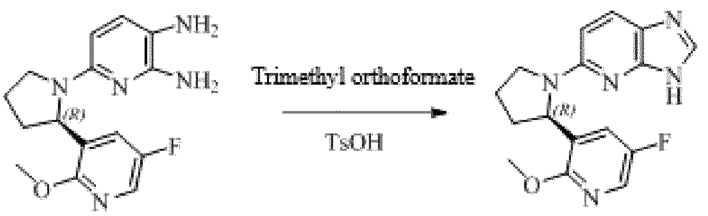


(R)-6-(2-(5-fluoro-2-methoxypyridin-3-yl)pyrrolidin-1-yl)pyridine-2,3-diamine (the crude product in the previous step, about 3.0 mmol) was dissolved in toluene (30 mL), trimethyl orthoformate (5.2 g, 49 mmol) and p-toluenesulfonic acid (170 mg, 0.99 mmol) were added to obtain a mixture. And the mixture was heated to 110°C, and reacted for 5 hours. Reaction solution was spin-dried, sodium bicarbonate solution (100 mL) and ethyl acetate (100 mL) were added, solution was separated, and the aqueous phase was extracted with ethyl acetate (50 mLx3). Organic phases were combined, and spin-dried, and it was purified by column chromatography (ethyl acetate), to obtain the target product (1.5 g, yield: 96.8%).

LC-MS (M/e) : 314.1 (M+H$^+$)

4. Preparation of (*R*)-3-(1-(3*H*-imidazo[4,5-b]pyridin-5-yl) pyrrolidin-2-yl) -5-fluoropyridin-2-ol

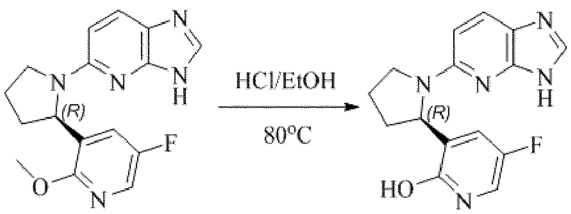


(*R*)-5-(2-(5-fluoro-2-methoxypyridin-3-yl)pyrrolidin-1-yl)-3H-imidazo[4,5-b]pyridine (1.5 g, 4.79 mmol) was added to hydrogen chloride ethanol solution (30 mL), and was heated to 90°C and reacted for 16 hours. Reaction solution was spin-dried, triethylamine (5 mL) was added to adjust pH to alkalinity, it was spin-dried, and purified by the column chromatography (dichloromethane: methanol=10:1), to obtain the target product (1.4 g, yield: 97.9%).

LC-MS (M/e) : 300.1 (M+H$^+$)

5. Preparation of tert-butyl ((*R*)-1-((3-((*R*)-1-(3H-imidazo[4,5-b]pyridin-5-yl)pyrrolidin-2-yl)-5-fluoropyridin-2-yl)oxy)propan-2-yl) carbamate

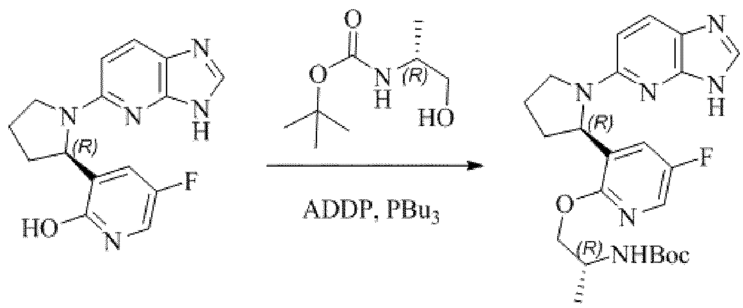


(*R*)-3-(1-(3*H*-imidazo[4,5-b]pyridin-5-yl)pyrrolidin-2-yl)-5-fluoropyridin-2-ol (700 mg, 2.34 mmol), tert-butyl (*R*)-(1-hydroxypropan-2-yl)carbamate (410 mg, 2.34 mmol) and tri-n-butylphosphine (945 mg, 4.68 mmol) were dissolved in tetrahydrofuran (10 mL), 1,1'-(azodicarbonyl)-dipiperidine (1.18 g, 4.68 mmol) was added under nitrogen protection to obtain a mixture. And the mixture was reacted for 2 h at 30°C. Reaction solution was concentrated, and purified by C18 column chromatography, to obtain the target product (500 mg, yield: 46.7%).

LC-MS (M/e) : 457.0 (M+H+)

6. Preparation of (*R*)-1-((3-((*R*)-1-(3*H*-imidazo[4,5-*b*]pyridin-5-yl)pyrrolidin-2-yl)-5-fluoropyridin-2-yl)oxy) propan-2-amine

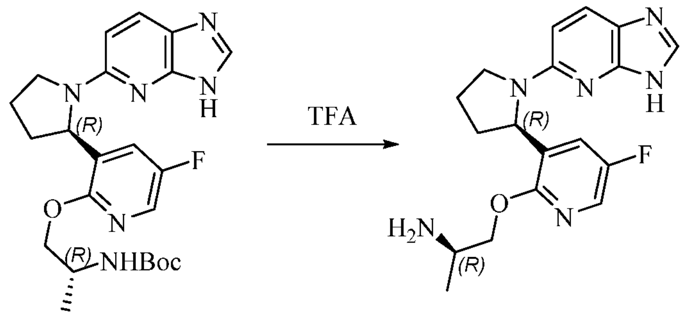


Tert-butyl ((*R*)-1-((3-((*R*)-1-(3*H*-imidazo[4,5-b]pyridin-5-yl)pyrrolidin-2-yl)-5-fluoropyridin-2-yl) oxy)propan-2-yl)carbamate (500 mg, 1.1 mmol) was dissolved in dichloromethane (20 mL), trifluoroacetic acid (20 mL) was added to obtain a mixture. And the mixture was reacted for 16 hours at 20°C. Reaction solution was concentrated, pH was adjusted to alkaline with the triethylamine, spin-dried, and purified by the C18 column chromatography, to obtain the crude target product (400 mg).

LC-MS (M/e) : 357.0 (M+H+)

7. Preparation of ($2^2$*R*,6*R*)-$3^5$-fluoro-6-methyl-$1^3$*H*-4-oxa-7-aza-1 (5,3)-imidazo[4,5-b]pyridina-3 (3,2)-pyridina-2(1,2)-pyrrolidinacyclooctaphan-8-one

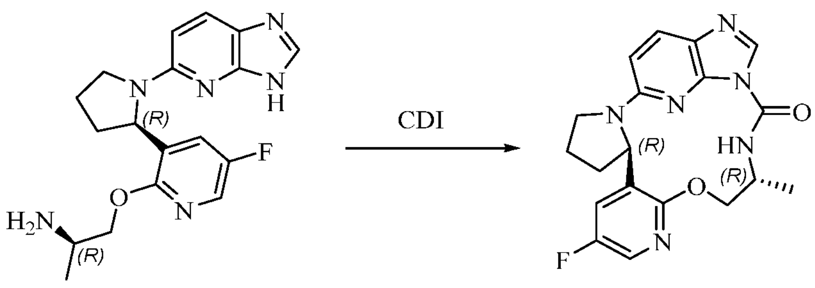


(*R*)-1-((3-((*R*)-1-(3*H*-imidazo[4,5-*b*]pyridin-5-yl)pyrrolidin-2-yl)-5-fluoropyridin-2-yl)oxy)propan-2-ami ne (the

crude product in the previous step, 400 mg, about 1.1 mmol) was dissolved in xylene (50 mL), carbonyl diimidazole (535 mg, 3.3 mmol) was added to obtain a mixture. And the mixture was heated to 130°C and reacted for 2 h. Reaction solution was concentrated, and purified by the column chromatography (petroleum ether: ethyl acetate=1:1), to obtain the crude target product (300 mg), and it was further separated by a high performance liquid chromatography (HPLC) high-pressure reverse phase preparation chromatography (acetonitrile: water=10%-70%), to obtain the title compound (100 mg, 23.8%).

Molecular formula: $C_{19}H_{19}FN_6O_2$, molecular weight: 382.4; LC-MS (M/e) : 383.2 ($M+H^+$).

$^1$H-NMR (400 MHz, $CDCl_3$) $\delta$: 10.50 (m, 1H), 8.36 (s, 1H), 7.86-7.90 (m, 2H), 7.31-7.35 (m, 1H), 6.51 (d, *J* = 8.8 Hz, 1H), 5.63-5.60 (m, 1H), 5.13-5.17 (m, 1H), 4.39-4.42 (m, 1H), 4.15-4.19 (m, 1H), 3.92-3.98 (m, 1H), 3.65-3.60 (m, 1H), 2.50-2.59 (m, 2H), 2.40-2.50 (m, 1H), 1.95-2.01 (m, 1H), 1.57(d, *J* = 6.8 Hz, 3H).

Embodiment 2: Preparation of crystal form II of the compound represented by Formula (I)

Preparation method I:

10.00 g of the compound represented by Formula (I) was mixed with 12.5 g of acetone, the mixture was stirred and heated to 50-55°C for dissolution and clarification. The mixture was cooled for crystallization, and continuously cooled to 10°C after a solid was separated out. 60 mL of water was dropped in the mixture at 10°C. When the dropping step was completed, it was stirred at a constant temperature for 0.5 h. Then the solution was suction-filtered, and vacuum-dried at 45-50°C, to obtain a product. The product was detected as the crystal form II of the compound represented by Formula (I) by XRD.

Preparation method II:

50.00 g of the compound represented by Formula (I) was mixed with 62.50 g of acetone, the mixture was stirred and heated to 50-55°C for dissolution and clarification. The mixture was cooled to 10-15°C for crystallization. 375 mL of n-heptane was dropped in the mixture at 10-15°C, and the mixture was stirred at a constant temperature, suction-filtered, and vacuum-dried at 50°C, to obtain a product. The product was detected as the crystal form II of the compound represented by Formula (I) by XRD.

Preparation method III:

3-1: 600.00 g of the compound represented by Formula (I) was mixed with 900 mL of an ethyl acetate, and the mixture was heated to 60-70°C for dissolution and clarification. The mixture was cooled to 20°C, and 6 L of n-heptane was dropped in the mixture at 20$\pm$5°C. The mixture was stirred at a constant temperature, suction-filtered, and vacuum-dried at 50°C, to obtain a product. The product was detected as the crystal form II of the compound represented by Formula (I) by XRD.

3-2: 1.45 kg of the compound represented by Formula (I) was mixed with 2.2 L of an ethyl acetate, and the mixture was heated to 60-70°C for dissolution and clarification. The mixture was controlled to 60-65°C, and n-heptane (15 L) was dropped in the mixture. The mixture was cooled to 10-20°C for crystallization, and stirred at a constant temperature, suction-filtered, and vacuum-dried at 45-50°C, to obtain a product. The product was detected as the crystal form II of the compound represented by Formula (I) by XRD.

XRPD test

X-ray reflection parameters: Cu, and K$\alpha$; entrance slit width: 0.6 mm; divergence slit width: 1 mm; scanning mode: continuous; scanning range: 3.0-45.0 degrees; sampling step length: 0.02 degrees; and scanning time of each step: 0.3 s.

The X-ray powder diffraction pattern of the crystal form II is shown in Fig. 1. The crystal form has peaks at the following diffraction $2\theta$ angles: 8.86$\pm$0.2°, 10.07$\pm$0.2°, 11.01 $\pm$0.2°, 12.54$\pm$0.2°, 13.43$\pm$0.2°, 14.13$\pm$0.2°, 15.06$\pm$0.2°, 15.96$\pm$0.2°, 16.70$\pm$0.2°, 18.66$\pm$ 0.2°, 19.10$\pm$0.2°, 19.71$\pm$0.2°, 20.42$\pm$0.2°, 21.60$\pm$0.2°, and 22.95$\pm$0.2°.

DSC test

The solid-state thermal properties of the crystal form II of the compound represented by Formula (I) are researched by DSC. DSC test conditions: it was purged with a nitrogen gas at 50 mL/min, data was collected at a heating rate of 3°C/min between 100°C and 200°C, and it was plotted under a condition that the endothermic peak faced downwards. The DSC curve of the crystal form II is shown in Fig. 2. The DSC thermogram shows that the crystal form

II has an endothermic transition peak at 160°C~165°C, and its maximum endothermic transition temperature (phase transition temperature), namely the temperature at the peak of the endothermic peak, is 162.25±3°C.

TGA analysis

TGA test conditions: it was purged with the nitrogen gas at 60 mL/min, and data was collected at a heating rate of 10°C/min between a room temperature and 350°C. The TGA curve of the crystal form II was shown in Fig. 3. The TGA pattern shows that the crystal form II has no apparent weight loss at 0°C- 250°C.
Experimental example of property test:

1. Stability test

Test sample: the crystal form II of the compound represented by Formula (I)

Test method: a suitable amount of the test samples was respectively weighed, and the appearance, moisture, content, related substances, XRD, and DSC of the test samples were investigated after being placed under the following conditions. The placing conditions were as follows.
Under the conditions of 60°C±2°C, or of 25 °C RH92.5%, it was placed for 10 days, opened/closed, and a sample was taken for testing on the 10th day (the opened package is a flat weighing bottle, and the closed package is a low-density polyethylene bag or low-density polyethylene bag + composite film bag.
Under the conditions of 40°C RH75%, it was placed for one month, opened/closed, and a sample was taken for testing after one month (the opened package is the flat weighing bottle, and the closed package is the low-density polyethylene bag or low-density polyethylene bag + composite film bag).
Moisture: it was determined according to the first 2 coulometric titration of the general rule 0832 (moisture determination) of the Chinese Pharmacopoeia (2015 Edition, Part IV).
XRD: it was determined according to the general rule 0451 (X-ray diffraction) of the Chinese Pharmacopoeia (2015 Edition, Part IV).
DSC: it was determined at a heating rate of 3°C/min, from 100°C to 200°C.
Related substances and content: it was determined according to the general rule 0512 (HPLC) of the Chinese Pharmacopoeia (2015 Edition, Part IV).
Experimental results:

Table 1: Stability investigation results of crystal form II

| Placing condition | | Total related substance (%) | Appearance | Moisture (%) | Content (%) | XRD | DSC (°C) |
|---|---|---|---|---|---|---|---|
| 0 day | | 0.09 | Off-white solid | 0.088 | 100.4 | -- | 162.32 |
| 60°C 10 days | Opened | 0.11 | Off-white solid | 0.18 | 99.8 | Same as 0 day | 162.24 |
| | Polyethylene bag | 0.11 | Off-white solid | 0.15 | 100.0 | Same as 0 day | 162.26 |
| | Polyethylene bag + composite film bag | 0.10 | Off-white solid | 0.13 | 99.7 | Same as 0 day | 162.23 |

(continued)

| Placing condition | | Total related substance (%) | Appearance | Moisture (%) | Content (%) | XRD | DSC (°C) |
|---|---|---|---|---|---|---|---|
| 25°C RH92.5% 10 days | Opened | 0.11 | Off-white solid | 0.13 | 99.6 | Same as 0 day | 162.23 |
| | Polyethylene bag | 0.10 | Off-white solid | 0.11 | 99.5 | Same as 0 day | 162.23 |
| | Polyethylene bag + composite film bag | 0.11 | Off-white solid | 0.11 | 99.3 | Same as 0 day | 162.27 |
| 40°C RH75% | Opened | 0.10 | Off-white solid | 0.026 | 100.8 | Same as 0 day | 162.29 |
| 1M | Polyethylene bag | 0.10 | Off-white solid | 0.033 | 101.0 | Same as 0 day | 162.18 |
| | Polyethylene bag + composite film bag | 0.10 | Off-white solid | 0.036 | 101.0 | Same as 0 day | 162.35 |

Experimental results:

Under the conditions of 60°C, opened/closed package, and placing for 10 days, or the conditions of 25°C RH92.5%, opened/closed package, and placing for 10 days, or the conditions of 40°C RH75%, opened/closed package, and placing for 1 month, the appearance, total related substances, content, moisture, XRD, DSC and the like of the samples are not changed, and the stability of the crystal form is good.

2. Hygroscopicity test of crystal form II

Test sample: the crystal form II of the compound represented by Formula (I).

Test method: it was determined according to the general rule 9103 (guidelines of drug hygroscopicity test) of the Chinese Pharmacopoeia (2015 Edition, Part IV) : the test sample was placed under the condition of 25°C and RH80% for 24 h (a sample bottle was placed under this condition for 24 h in advance), and results were shown in Table 2:

Table 2: Investigation results of hygroscopicity

| Test sample | Placing condition | Moisture absorption weight gain (%) |
|---|---|---|
| Crystal form II | 25°C RH80% placing for 1 day | 0.002 |

Experimental results: this product has no or almost no hygroscopicity.

Activity test

An exemplary activity test of the compound of the present disclosure is provided below, to show the beneficial activities and beneficial technical effects of the compound of the present disclosure. However, it should be understood that the following experimental scheme is only an example of the content of the present disclosure, not limitation to a scope of protection of the present disclosure.

Experimental example 1: In vitro cytological inhibitory activity of compound of disclosure

Experimental materials

Test compound: the compound represented by Formula (I), prepared according to the method in Embodiment 1.
The meanings of cell lines used in the experiment are as follows:

Ba/F3 LMNA-NTRK1-G595R cell line:

A stably expressed cell line of Ba/F3 cells transfected with LMNA-NTRK1 G595R; and

Ba/F3 ETV6-NTRK3-G623R cell line:

A stably expressed cell line of Ba/F3 cells transfected with ETV6-NTRK3-G623R.

Experimental method (CelltiterGlo assay)

1. Cell culture and inoculation

All cells were suspension cells, and all culture mediums were RPMI-1640+10% FBS. The cells were tested in a logarithmic growth phase.
The cells in the logarithmic growth phase were collected and counted by a platelet counter. A trypan blue exclusion method was used to detect the cell activity, to ensure that the cell activity was above 90%. It was adjusted to the appropriate concentration, and 90 μL of cell suspension was respectively added to a 96-well plate.

Table 3: Number of cells inoculated

| Cell line | Number of cells inoculated |
|---|---|
| Ba/F3 LMNA-NTRK1-G595R | 3000 cells/well |
| Ba/F3 ETV6-NTRK3-G623R | 3000 cells/well |

2. Preparation of test compound

"M" means "mol/L", " mM "means "mmol/L", and "μM" means "μmol/L".
2.1 Dimethylsulfoxide (DMSO) stock solution of the test compound was prepared, and the concentration was shown in Table 4.

Table 4: Stock solution concentration of test compound (mM)

| Compound | Stock solution concentration |
|---|---|
| Compound represented by Formula (I) | 10 mM |

2.2 Preparation of working stock solution of test compound

The stock solution of the test compound was diluted from 10 mM to 1 mM with DMSO, and then diluted by three times in a continuous gradient with DMSO. There were a total of 9 gradients. 2 μL of compound solution gradient-diluted with DMSO was respectively taken and added to 198 μL of culture solution, to obtain the working stock solution with the test compound (the concentration of the compound was 10 times of the final concentration, the concentration of DMSO was 1wt%, and the maximum concentration was 10 μM).
The maximum value was a DMSO solvent control, only the culture medium was added to a blank well, and the cells were not inoculated.

2.3 Compound treatment

10 μL compound working stock solution (10 times of dilution, and the DMSO final concentration was 0.1%) was added to each well of the 96-well plate inoculated with the cells.

The final concentration of the test compound was: 1000 nM, 333.33 nM, 111.11 nM, 37.04 nM, 12.35 nM, 4.12 nM, 1.37 nM, 0.46 nM, and 0.15 nM.
It was incubated in a 5% $CO_2$ cell incubator for 72 hours.

3. Detection

A CellTiter-Glo luminescent cell viability assay (CTG) reagent was molten and a cell plate was balanced to a room temperature for 30 minutes. 60 μL of a reagent (Celltiter Glass assay kit) was added to each well, it was shaken with an oscillator for 2 min to be mixed uniformly (avoiding light), and incubated at the room temperature for 10 min (avoiding light). The light signal value was read by a multifunctional microplate reader.

4. Data processing

1) Inhibition rate (%) = (DMSO solvent control well reading - test compound well reading) / (DMSO solvent control well reading - blank well reading) × 100 %; and

2) it was input into GraphPad Prism 5.0 for drawing, to obtain a curve and $IC_{50}$.

Experimental results and conclusions

Table 5: In vitro cytological activity of compound of present disclosure ($IC_{50}$, nM)

| Test compound | Ba/F3 LMNA-NTRK1-G595R (nM) | Ba/F3 ETV6-NTRK3-G623R (nM) |
|---|---|---|
| Compound represented by Formula (I) | 0.6 | 1 |

It may be seen from Table 5 that the compound of the present disclosure may effectively inhibit the proliferation of cells such us Ba/F3 LMNA-NTRK1-G595R and Ba/F3 ETV6-NTRK3-G623R. It is indicated that the compound of the present disclosure has clinical application potential in treating drug-resistant cancererous diseases caused by a neurotrophin receptor kinase (NTRK) gene mutation.

Experimental example 2: In vitro cytological inhibitory activity of compound of present disclosure

Experimental materials

Test compound: the compound represented by Formula (I), prepared according to the method in Embodiment 1.
The meanings of cell lines used in the experiment are as follows:
Ba/F3 SLC34A2/ROS1-G2032R cell line:
A stably expressed cell line of Ba/F3 cells transfected with SLC34A2/ROS1-G2032R.

Experimental method (Celltiter-Glo assay)

1. Cell culture and inoculation

All cells were suspension cells, and all culture mediums were RPMI-1640+10% FBS. The cells were tested in a logarithmic growth phase.
The cells in the logarithmic growth phase were collected and counted by a platelet counter. A trypan blue exclusion method was used to detect the cell activity, as to ensure that the cell activity was above 90%. It was adjusted to the appropriate concentration, and 90 μL of cell suspension was respectively added to a 96-well plate.

Table 6: Number of cells inoculated

| Cell line | Number of cells inoculated |
|---|---|
| Ba/F3 SLC34A2/ROS1-G2032R | 3000 cells/well |

2. Preparation of test compound

2.1 DMSO stock solution with the test compound was prepared, and the concentration was shown in Table 7.

Table 7: Stock solution concentration of test compound (mM)

| Compound | Stock solution concentration |
|---|---|
| Compound represented by Formula (I) | 10 mM |

2.2 Preparation of working stock solution of test compound

The stock solution of the test compound was diluted from 10 mM to 1 mM with DMSO, and then diluted by 3.16 times in a continuous gradient with DMSO, and there were a total of 9 gradients. 2 μL of compound solution gradient-diluted with DMSO was respectively taken and added to 198 μL of culture solution, to obtain the working stock solution of the test compound. In the working stock solution, the concentration of the compound was 10 times of the final concentration, the concentration of DMSO was 1%, and the maximum concentration was 10 μM.

The maximum value was a DMSO solvent control, only the culture medium was added to a blank well, and the cells were not inoculated.

2.3 Compound treatment

10 μL of the compound working stock solution (10 times of dilution, and the DMSO final concentration was 0.1 %) was added to each well of the 96-well plate inoculated with the cells.

The final concentration of the test compound was: 1000 nM, 316 nM, 100 nM, 31.6 nM, 10 nM, 3.16 nM, 1 nM, 0.316 nM, and 0.1 nM.

It was incubated in a 5% $CO_2$ cell incubator for 72 hours.

3. Detection

A CTG reagent was molten and a cell plate was balanced to a room temperature for 30 minutes. 100 μL of a reagent (Celltiter-Glass assay kit) was added to each well, it was shaken with an oscillator for 5 min to be mixed uniformly (avoiding light), and incubated at the room temperature for 20 min (avoiding light). The light signal value was read by a multifunctional microplate reader.

4. Data processing

1) Cell survival rate (%) = (test compound well reading - blank well reading) / (DMSO solvent control well reading - blank well reading) × 100 %; and

2) it was input into GraphPad Prism 5.0 for drawing, to obtain a curve and $IC_{50}$.

Experimental results and conclusions

Table 8: In vitro cytological activity of compound of present disclosure ($IC_{50}$, nM)

| Test compound | Ba/F3 SLC34A2/ROS1-G2032R |
|---|---|
| Compound represented by Formula (I) | 2.3 |

It may be seen from Table 8 that the compound of the present disclosure may effectively inhibit the proliferation of Ba/F3 SLC34A2/ROS1-G2032R cells. It is indicated that the compound of the present disclosure has clinical application potential in treating drug-resistant cancer diseases caused by a ROS gene mutation.

Experimental example 3: Efficacy study experiment in compound of the present invention on BaF3 LMNA-NTRK1-G595R stable cell strain subcutaneous xenograft model.

Test substance: the compound represented by Formula (I), prepared according to the method in Embodiment 1.

Positive control drug: a compound LOXO-195, purchased or prepared according to a method disclosed in WO2011146336, and its structure is shown as follows:

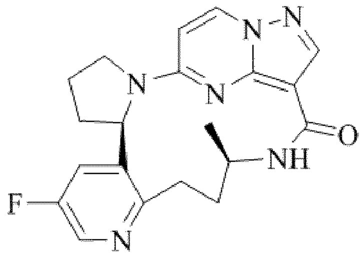

Experimental method

3.1 Cell inoculation

A BaF3LMNA-NTRK1-G595R stably transfected cell line resuspended in a RPMI1640 serum-free medium was inoculated subcutaneously on a right scapula of a mouse (nonobese diabetic-severe combined immunodeficiency disease (NOD-SCID) ) at $1\times10^6$ cells/mouse (0.1 ml/mouse).

3.2 Grouping administration

While the average tumor volume was about 500 $mm^3$, it was randomly divided into a vehicle group, a compound represented by Formula (I) (10/5/3/1 mg/kg, bid) group and a LOXO-195 group (30/10/3 mg/kg, bid).

The detailed administration method, administration dosage and administration route were shown in Table 9.

Table 9-1: Administration route, dosage and scheme of BaF3LMNA-NTRK1-G595R model efficacy experiment 1

| Group | n | Administration group | Dosage (mg/Kg) | Administration mode | Administration time |
|---|---|---|---|---|---|
| 1 | 6 | Vehicle group | - | - | - |
| 2 | 6 | LOXO-195 group | 3 | p.o.,bid | 7 days |
| 3 | 6 | LOXO-195 group | 10 | p.o.,bid | 7 days |
| 4 | 6 | Compound represented by Formula (I) | 3 | p.o.,bid | 7 days |

Table 9-2: Administration route, dosage and scheme of BaF3 LMNA-NTRK1-G595R model efficacy experiment 2

| Group | n | Administration group | Dosage (mg/Kg) | Administration mode | Administration time |
|---|---|---|---|---|---|
| 5 | 6 | LOXO-195 group | 30 | p.o.,bid | 7 days |
| 6 | 6 | Compound represented by Formula (I) | 1 | p.o.,bid | 7 days |
| 7 | 6 | Compound represented by Formula (I) | 5 | p.o.,bid | 7 days |
| 8 | 6 | Compound represented by Formula (I) | 10 | p.o.,bid | 7 days |
| Note: n: number of animals; and the drug was administered on the day of grouping. | | | | | |

3.3 Experimental evaluation index

1) Tumor growth inhibition rate TGI (%)

$$TGI\ (\%)=(1-T/C)\times 100\%$$

2) Tumor volume ratio of Treatment group/Control group T/C (%)

$$T/C\ (\%)=T_{RTV}/C_{RTV}\times 100\%$$

*$RTV=Vt/V_0$, herein Vt is the tumor volume on the t-th day after grouping, and $V_0$ is the tumor volume on the day of grouping; $T_{RTV}$: the average relative tumor volume in the administration group; and $C_{RTV}$: the average relative tumor volume in the vehicle control group.

3) Efficacy evaluation standard: T/C (%)>40% is non-effective, and T/C (%)≤40% is effective; and tumor growth inhibition rate ≥60% is effective.

3.4 Experimental results

Table 10-1: Pharmacodynamic experiment 1 inhibitory effect on xenograft tumor of BaF3 LMNA-NTRK1-G595R stably transfected cell line

| | Group | Administration dosage (mg/Kg) | T/C (%) | TGI (%) |
|---|---|---|---|---|
| Group 1 | Vehicle | | - | - |
| Group 2 | LOXO-195 | 3, bid | 93.6 | 6.4 |
| Group 3 | LOXO-195 | 10, bid | 63.1 | 36.9 |
| Group 4 | Compound represented by Formula (I) | 3, bid | 73.2 | 26.8 |

Table 10-2: Pharmacodynamic experiment 2 inhibitory effect on xenograft tumor of BaF3 LMNA-NTRK1-G595R stably transfected cell line

| | Group | Administration dosage (mg/Kg) | T/C (%) | TGI (%) |
|---|---|---|---|---|
| Group 5 | LOXO-195 | 30, bid | 0 | 100 |
| Group 6 | Compound represented by Formula (I) | 1, bid | 53.7 | 46.3 |
| Group 7 | Compound represented by Formula (I) | 5, bid | 0 | 100 |
| Group 8 | Compound represented by Formula (I) | 10, bid | 0 | 100 |

Experimental conclusions

Experimental data show that the oral administration of the compound of the present disclosure may significantly inhibit the tumor efficacy model of cell line BaF3LMNA-NTRK1-G595R containing the NTRK fusion gene. In other words,

the compound of the present disclosure has the good tumor inhibition effect on tumors with the NTRK fusion gene mutation, and has a good clinical application prospect.

In addition to those described herein, according to the foregoing descriptions, various modifications of the present disclosure may be apparent to those skilled in the art. Such modifications are also intended to fall within the scope of the appended claims. All reference documents cited in the present application (including all patents, patent applications, journal articles, books and any other publications) are incorporated herein by reference in its entirety.

## Claims

1. A crystal form II of a compound represented by Formula (I), having characteristic peaks at angles (2θ) 10.07±0.2°, 11.01±0.2°, 12.54±0.2°, 14.13±0.2°, 15.96±0.2°, 16.70±0.2° and 21.60±0.2° in an X-ray powder diffraction pattern using Cu-Kα radiation,

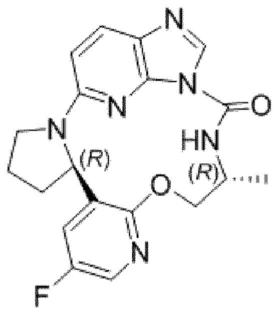

(I).

2. The crystal form II according to claim 1, futher having characteristic peaks at 8.86±0.2°, 13.43±0.2°, 15.06±0.2°, 18.66±0.2°, 19.10±0.2°, 19.71±0.2°, 20.42±0.2° and 22.95±0.2° in the X-ray powder diffraction pattern.

3. The crystal form II according to claim 1 or 2, wherein the X-ray powder diffraction pattern is basically as shown in Fig. 1.

4. The crystal form II according to any one of claims 1-3, wherein a differential scanning calorimetry thermogram shows that it has an endothermic transition peak at 155°C -170 °C ; preferably, it has an endothermic transition peak at 160°C -165 °C ; preferably, the transition temperature at which it generates a maximum endothermic quantity is 162.25±3°C; and preferably, a differential scanning calorimetry thermogram of the crystal form II is basically as shown in Fig. 2.

5. A method for preparing the crystal form II according to any one of claims 1-4 comprising: mixing the compound represented by Formula (I) with an organic solvent, heating until the compound is completely dissolved, dropping a poor solvent, separating out a solid, filtering and drying, to obtain the crystal form II.

6. The preparation method for the crystal form II according to claim 5, comprising one or more of the followings:

   (1) the organic solvent is selected from one or more of acetone and ethyl acetate;
   (2) the poor solvent is selected from one or more of water and n-heptane; and
   (3) it is heated to 30°C-80°C, preferably heated to 40°C-70°C, preferably heated to 40-45°C, preferably heated to 45-50°C, preferably heated to 50-55°C, preferably heated to 55-60°C, preferably heated to 60-65°C, preferably heated to 65-70°C, and more preferably heated until the compound is dissolved and becomes clear.

7. A pharmaceutical formulation or a pharmaceutical composition, wherein it contains the crystal form II of the compound represented by Formula (I) according to any one of claims 1-4, and one or more pharmaceutically acceptable carriers and/or excipients.

8. The pharmaceutical composition according to claim 7, wherein it further contains one or more second therapeutic active agents selected from the following:

   a drug for treating pain; preferably, it is selected from a Nav1.7 channel regulator, an opioid analgesic, a non-steroidal anti-inflammatory drug, a sedative, a selective/non-selective cyclooxygenase inhibitor, an antiepileptic

drug, an antidepressant, a local anesthetic, a 5-HT receptor blocker, a 5-HT receptor agonist, an ergot alkaloid, a β-receptor blocker, an M receptor blocker, a nitrate, and a vitamin K;
a drug for treating cancer; preferably, it is selected from a mitotic inhibitor, an alkylating agent, an antimetabolite, an antisense deoxyribonucleic acid or ribonucleic acid, an anti-tumor antibiotic, a growth factor inhibitor, a signal transduction inhibitor, a cell cycle inhibitor, an enzyme inhibitor, a retinoid receptor modulator, a proteasome inhibitor, a topoisomerase inhibitor, a biological response modifier, a hormone drug, an angiogenesis inhibitor, a cell growth inhibitor, a targeting antibody, an HMG-CoA reductase inhibitor, and an isoprene protein transferase inhibitor;
a drug for treating neurodegenerative diseases; preferably, it is selected from a dopaminergic drug, a dopamine receptor agonist, a dopamine metabolism-affecting drug, an N-methyl-D-aspartate receptor antagonist, an adenosine $A_{2A}$ receptor inhibitor, a DA releasing and reuptake-affecting drug, a central anticholinergic drug, a cholinesterase inhibitor, a 5-HT agonist, an α2 adrenergic receptor antagonist, an antidepressant, a choline receptor agonist, a β/γ secretase inhibitor, an H3 receptor antagonist and an antioxidant drug;
a drug for treating autoimmune diseases; preferably, it is selected from an antirheumatic drug for improving conditions, a non-steroidal anti-inflammatory drug, a glucocorticoid drug, a tumor necrosis factor antagonist, a cyclophosphamide, a mycophenolate mofetil and a cyclosporin; and
a drug for treating inflammation; preferably, it is selected from a steroidal anti-inflammatory drug and a non-steroidal anti-inflammatory drug.

**9.** A use of the crystal form II of the compound represented by Formula (I) according to any one of claims 1-4, the pharmaceutical formulation according to claim 7, or the pharmaceutical composition according to claim 8 in preparing a drug for treating and/or preventing diseases and related diseases mediated by one or more tyrosine kinases in a tropomyosin receptor kinase, an anaplastic lymphoma kinase and/or a c-ros oncogene 1 receptor kinase, wherein the diseases and related diseases are selected from one or more of pain, cancer, inflammation, neurodegenerative diseases and autoimmune diseases.

**10.** A method for treating and/or preventing diseases and related diseases mediated by one or more tyrosine kinases in a tropomyosin receptor kinase, an anaplastic lymphoma kinase and/or a c-ros oncogene 1 receptor kinase, comprising steps of administering to a subject in need an effective amount of the crystal form II of the compound represented by Formula (I) according to any one of claims 1-4, the pharmaceutical formulation according to claim 7, or the pharmaceutical composition according to claim 8.

**11.** The use according to claim 9 or the method according to claim 10, wherein the disease mediated by one or more tyrosine kinases in a tropomyosin receptor kinase, an anaplastic lymphoma kinase and/or a c-ros oncogene 1 receptor kinase is a cancer, and the cancer is selected from lung cancer, colon cancer, rectal cancer, prostate cancer, breast cancer, liver cancer, gallbladder cancer, cholangiocarcinoma, leukemia, melanoma, lymphatic cancer, thyroid cancer, multiple myeloma, soft tissue sarcoma, ovarian cancer, cervical cancer, fallopian tube carcinoma, renal cell carcinoma, gastric cancer, gastrointestinal stromal tumor, bone cancer, basal cell cancer, peritoneal cancer, dermatofibroma, pancreatic cancer, esophageal cancer, glioblastoma, head and neck cancer, inflammatory myofibroblast tumor, anaplastic large cell lymphoma and neuroblastoma; and
preferably, the lung cancer is selected from small cell lung cancer and non-small cell lung cancer.

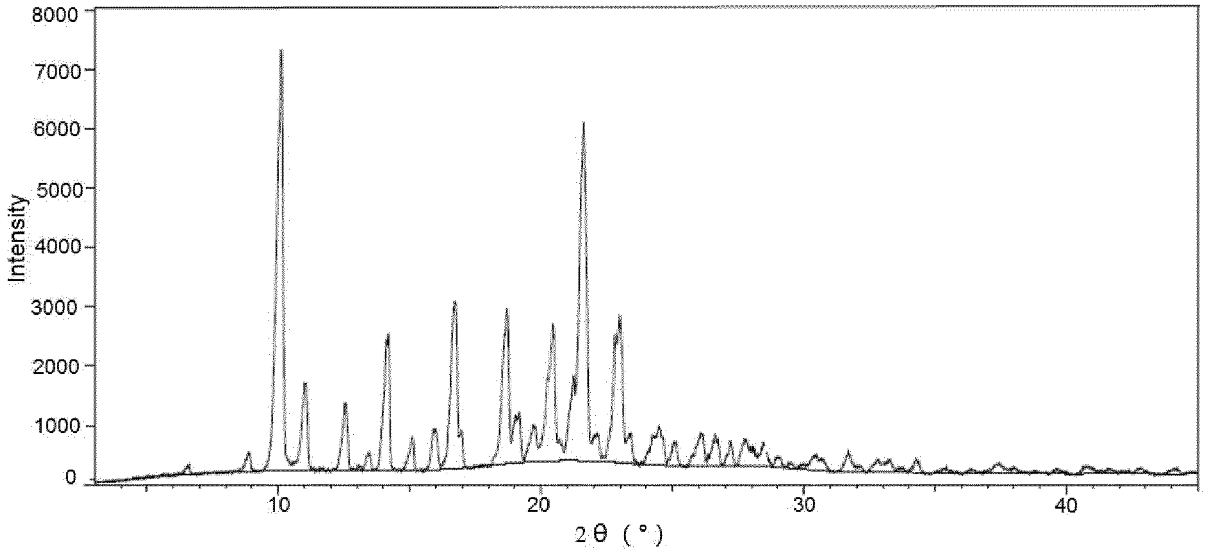
8000
7000
6000
5000
4000
3000
2000
1000
0
Intensity
10
20
30
40
2θ (°)

Fig. 1

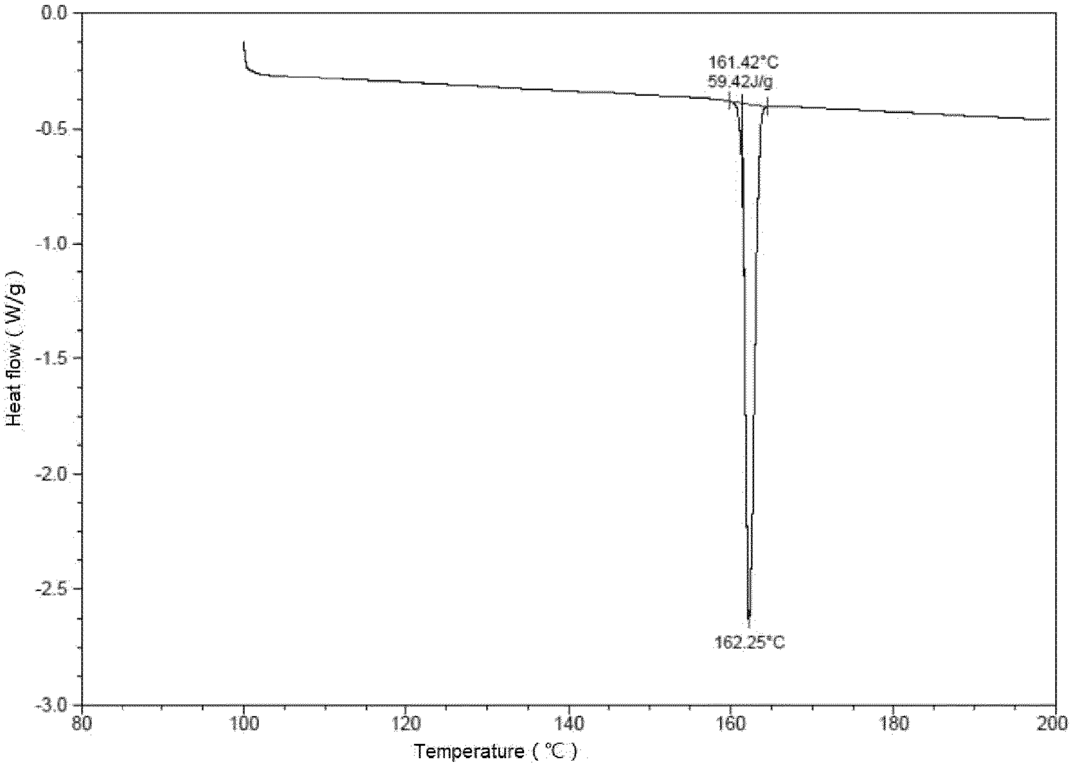
161.42°C
59.42J/g
162.25°C
Heat flow ( W/g )
Temperature ( ℃ )
0.0
-0.5
-1.0
-1.5
-2.0
-2.5
-3.0
80
100
120
140
160
180
200

Fig. 2

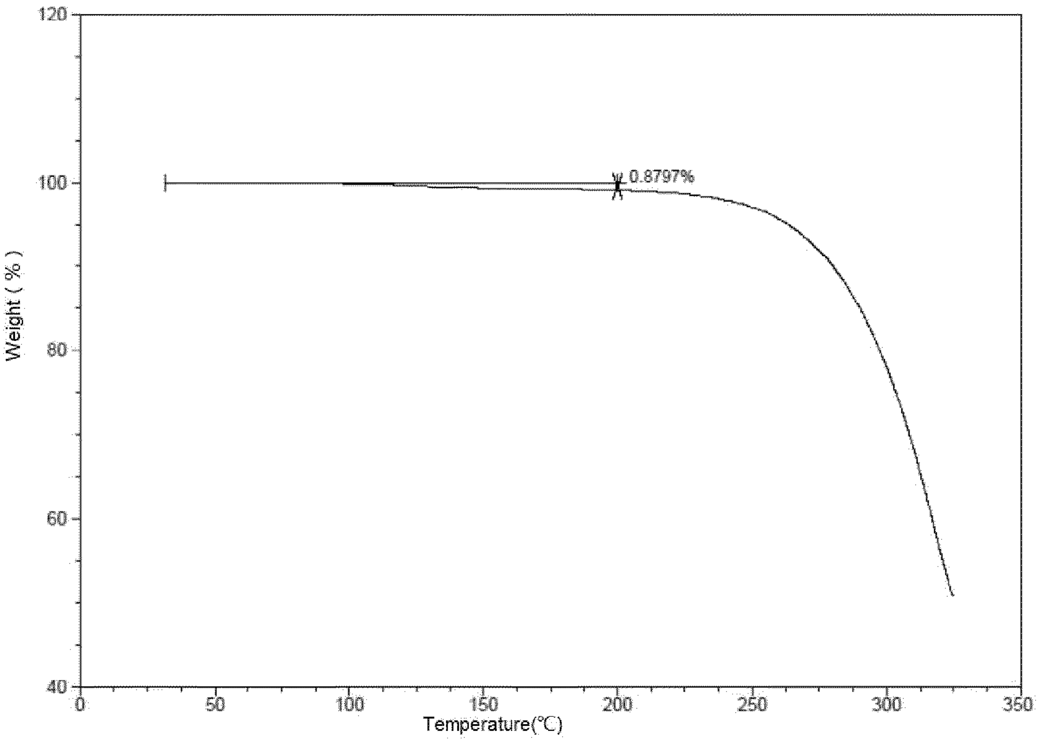
0.8797%
Weight ( % )
Temperature(℃)
120
100
80
60
40
0
50
100
150
200
250
300
350

Fig. 3

| INTERNATIONAL SEARCH REPORT | International application No.<br>**PCT/CN2021/092225** |
|---|---|

**A. CLASSIFICATION OF SUBJECT MATTER**

C07D 487/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D

Documentation searched other than minimum documentation to the extent that such documents are included in the fields searched

Electronic data base consulted during the international search (name of data base and, where practicable, search terms used)

REGISTRY, CAPLUS, DWPI, CNABS, CNKI: 依据式（I）的结构进行了检索, search based on the structural of formula (I), 轩竹医药, LOXO-195, 刘斌, 大环, 激酶, macrocyclic, kinase, inhibit

**C. DOCUMENTS CONSIDERED TO BE RELEVANT**

| Category* | Citation of document, with indication, where appropriate, of the relevant passages | Relevant to claim No. |
|---|---|---|
| PX | WO 2020094112 A1 (SHANDONG XUANZHU PHARMA CO., LTD.) 14 May 2020 (2020-05-14)<br>compound 3 | 1-11 |
| A | WO 2015112806 A2 (TP THERAPEUTICS, INC.) 30 July 2015 (2015-07-30)<br>compounds 20A, 20B and 20 | 1-11 |
| A | WO 2019094143 A1 (ANGEX PHARMACEUTICAL INC.) 16 May 2019 (2019-05-16)<br>entire document | 1-11 |
| A | CN 103748099 A (CENTRO NACIONAL DE INVESTIGACIONES ONCOLOGICAS (CNIO)) 23 April 2014 (2014-04-23)<br>entire document | 1-11 |
| A | WO 2010028116 A1 (MERCK SERONO SA) 11 March 2010 (2010-03-11)<br>entire document | 1-11 |
| A | WO 2011146336 A1 (ARRAY BIOPHARMA, INC.) 24 November 2011 (2011-11-24)<br>embodiment 33, and table 1 | 1-11 |

☐ Further documents are listed in the continuation of Box C.　　☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **16 June 2021** | **29 June 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/092225**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10-11**
because they relate to subject matter not required to be searched by this Authority, namely:

[1] Claims 10 and 11 relate to methods of treatment performed on a human or animal body (PCT Rule 39.1(iv)). Nevertheless, a search is made on the basis of the technical subject of the use of said compounds/compositions in the preparation of the corresponding drugs.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/092225**

| Patent document cited in search report | Publication date (day/month/year) | Patent family member(s) | Publication date (day/month/year) |
|---|---|---|---|
| WO 2020094112 A1 | 14 May 2020 | None | |
| WO 2015112806 A2 | 30 July 2015 | IL 246860 A | 26 September 2019 |
| | | CN 106170289 A | 30 November 2016 |
| | | WO 2015112806 A3 | 12 November 2015 |
| | | CN 106170289 B | 23 July 2019 |
| | | US 2017002023 A1 | 05 January 2017 |
| | | AP 2016009383 A0 | 31 August 2016 |
| | | SG 10202000191Y A | 30 March 2020 |
| | | JP 2017503867 A | 02 February 2017 |
| | | CL 2016001876 A1 | 03 February 2017 |
| | | EA 031863 B1 | 29 March 2019 |
| | | US 2017334929 A1 | 23 November 2017 |
| | | AP 201609383 A0 | 31 August 2016 |
| | | LT 3097107 T | 25 July 2019 |
| | | US 2019169207 A1 | 06 June 2019 |
| | | EP 3097107 A4 | 09 August 2017 |
| | | SG 11201605929R A | 30 August 2016 |
| | | HR P20191283 T1 | 18 October 2019 |
| | | ZA 201604654 B | 28 October 2020 |
| | | CN 110317214 A | 11 October 2019 |
| | | SI 3097107 T1 | 30 August 2019 |
| | | AU 2019279951 A1 | 16 January 2020 |
| | | DK 3097107 T3 | 08 July 2019 |
| | | PT 3097107 T | 19 July 2019 |
| | | EP 3097107 A2 | 30 November 2016 |
| | | EP 3636649 A1 | 15 April 2020 |
| | | US 2020216465 A1 | 09 July 2020 |
| | | KR 20160111395 A | 26 September 2016 |
| | | AU 2015209239 B2 | 12 September 2019 |
| | | PE 20160931 A1 | 21 September 2016 |
| | | PH 12016501463 A1 | 06 February 2017 |
| | | IL 268820 D0 | 31 October 2019 |
| | | EP 3636648 A1 | 15 April 2020 |
| | | PL 3097107 T3 | 31 January 2020 |
| | | ES 2735729 T3 | 20 December 2019 |
| | | EP 3636650 A1 | 15 April 2020 |
| | | IL 246860 D0 | 31 August 2016 |
| | | HU E045208 T2 | 30 December 2019 |
| | | MX 2016009588 A | 15 May 2017 |
| | | EP 3572416 A1 | 27 November 2019 |
| | | EA 201892241 A1 | 28 February 2019 |
| | | US 10618912 B2 | 14 April 2020 |
| | | US 9714258 B2 | 25 July 2017 |
| | | JP 6490713 B2 | 27 March 2019 |
| | | RS 59059 B1 | 30 August 2019 |
| | | EA 201691492 A1 | 30 June 2017 |
| | | CA 2936079 A1 | 30 July 2015 |
| | | CN 110317213 A | 11 October 2019 |
| | | EP 3097107 B1 | 17 April 2019 |
| | | HK 1231407 A1 | 22 December 2017 |

Form PCT/ISA/210 (patent family annex) (January 2015)

**INTERNATIONAL SEARCH REPORT**
**Information on patent family members**

International application No.
**PCT/CN2021/092225**

| Patent document cited in search report | Publication date (day/month/year) | Patent family member(s) | Publication date (day/month/year) |
|---|---|---|---|
| WO 2019094143 A1 | 16 May 2019 | CN 111343987 A | 26 June 2020 |
| | | EP 3706749 A1 | 16 September 2020 |
| | | TW 201927790 A | 16 July 2019 |
| | | JP 2021502413 A | 28 January 2021 |
| | | US 2020385386 A1 | 10 December 2020 |
| | | KR 20200085830 A | 15 July 2020 |
| | | EP 3706749 A4 | 03 March 2021 |
| | | AU 2018364938 A1 | 04 June 2020 |
| | | CA 3081790 A1 | 16 May 2019 |
| CN 103748099 A | 23 April 2014 | MX 349366 B | 26 July 2017 |
| | | CA 2872979 C | 18 February 2020 |
| | | KR 20140043092 A | 08 April 2014 |
| | | US 9808466 B2 | 07 November 2017 |
| | | US 2014256717 A1 | 11 September 2014 |
| | | BR 112013029711 A2 | 24 January 2017 |
| | | NZ 618157 A | 31 July 2015 |
| | | CA 2872979 A1 | 22 November 2012 |
| | | MX 2013013462 A | 27 February 2014 |
| | | EP 2710018 A2 | 26 March 2014 |
| | | WO 2012156756 A3 | 10 January 2013 |
| | | US 2016296528 A1 | 13 October 2016 |
| | | ZA 201309128 B | 27 August 2014 |
| | | SG 11201400681U A | 28 August 2014 |
| | | RU 2598840 C2 | 27 September 2016 |
| | | AU 2012257513 B2 | 11 May 2017 |
| | | WO 2012156756 A2 | 22 November 2012 |
| | | CN 103748099 B | 17 August 2016 |
| | | KR 101953210 B1 | 28 February 2019 |
| | | JP 5997763 B2 | 28 September 2016 |
| | | BR 112013029711 B1 | 12 May 2020 |
| | | JP 2014515346 A | 30 June 2014 |
| | | RU 2013156361 A | 27 June 2015 |
| | | AU 2012257513 A1 | 12 December 2013 |
| | | US 9284334 B2 | 15 March 2016 |
| WO 2010028116 A1 | 11 March 2010 | ES 2668834 T3 | 22 May 2018 |
| | | CN 102143750 B | 02 April 2014 |
| | | AU 2009288021 A1 | 11 March 2010 |
| | | US 8815872 B2 | 26 August 2014 |
| | | CN 102143750 A | 03 August 2011 |
| | | EA 201100450 A1 | 31 October 2011 |
| | | US 2011294801 A1 | 01 December 2011 |
| | | CA 2735420 A1 | 11 March 2010 |
| | | IL 211584 A | 30 April 2017 |
| | | HK 1158947 A1 | 27 July 2012 |
| | | EP 2334301 B1 | 14 February 2018 |
| | | JP 5749645 B2 | 15 July 2015 |
| | | AU 2009288021 B2 | 18 June 2015 |
| | | JP 2012502043 A | 26 January 2012 |
| | | CA 2735420 C | 28 June 2016 |
| | | AR 073551 A1 | 17 November 2010 |

Form PCT/ISA/210 (patent family annex) (January 2015)

**INTERNATIONAL SEARCH REPORT**
**Information on patent family members**

International application No.
**PCT/CN2021/092225**

| Patent document cited in search report | Publication date (day/month/year) | Patent family member(s) | Publication date (day/month/year) |
|---|---|---|---|
| | | MX 2011002470 A | 05 April 2011 |
| | | EP 2334301 A1 | 22 June 2011 |
| | | ZA 201101243 B | 30 May 2012 |
| | | KR 20110073500 A | 29 June 2011 |
| WO 2011146336 A1 | 24 November 2011 | AU 2018208676 B2 | 30 July 2020 |
| | | NZ 604708 A | 29 May 2015 |
| | | AU 2011256380 C1 | 08 September 2016 |
| | | BR 112012029405 A8 | 17 December 2019 |
| | | US 9750744 B2 | 05 September 2017 |
| | | US 9840519 B2 | 12 December 2017 |
| | | US 9493476 B2 | 15 November 2016 |
| | | SI 3205654 T1 | 31 July 2019 |
| | | BR 112012029405 B1 | 05 January 2021 |
| | | US 2020291026 A1 | 17 September 2020 |
| | | DK 3205654 T3 | 15 April 2019 |
| | | KR 20130086951 A | 05 August 2013 |
| | | CY 1119345 T1 | 14 February 2018 |
| | | DK 2918588 T3 | 28 August 2017 |
| | | KR 101852169 B1 | 26 April 2018 |
| | | AU 2016203348 C1 | 12 November 2020 |
| | | ME 03376 B | 20 January 2020 |
| | | US 2017107232 A1 | 20 April 2017 |
| | | RU 2016128920 A | 07 December 2018 |
| | | AU 2011256380 A1 | 10 January 2013 |
| | | JP 5832527 B2 | 16 December 2015 |
| | | JP 2017082018 A | 18 May 2017 |
| | | US 2017114059 A1 | 27 April 2017 |
| | | ES 2628418 T3 | 02 August 2017 |
| | | EP 2571883 B1 | 07 January 2015 |
| | | TW 201936197 A | 16 September 2019 |
| | | UY 33395 A | 30 November 2012 |
| | | EP 3521291 A1 | 07 August 2019 |
| | | ES 2534335 T3 | 21 April 2015 |
| | | CO 6660502 A2 | 30 April 2013 |
| | | SI 2918588 T1 | 29 September 2017 |
| | | MX 365251 B | 28 May 2019 |
| | | AU 2016203348 A1 | 16 June 2016 |
| | | LT 2918588 T | 25 August 2017 |
| | | KR 20190101498 A | 30 August 2019 |
| | | EP 3205654 B1 | 02 January 2019 |
| | | HR P20171140 T1 | 06 October 2017 |
| | | RU 2012155278 A | 27 June 2014 |
| | | SG 10201506593X A | 29 October 2015 |
| | | AR 081919 A1 | 31 October 2012 |
| | | HU E035337 T2 | 02 May 2018 |
| | | PH 12015500190 B1 | 19 October 2015 |
| | | RU 2594742 C2 | 20 August 2016 |
| | | PL 3205654 T3 | 30 August 2019 |
| | | KR 20200085364 A | 14 July 2020 |
| | | RS 58537 B1 | 30 April 2019 |

Form PCT/ISA/210 (patent family annex) (January 2015)

**INTERNATIONAL SEARCH REPORT**
**Information on patent family members**

International application No.
**PCT/CN2021/092225**

| Patent document cited in search report | Publication date (day/month/year) | Patent family member(s) | Publication date (day/month/year) |
|---|---|---|---|
| | | ES 2718043 T3 | 27 June 2019 |
| | | TW 201202254 A | 16 January 2012 |
| | | EP 2918588 A1 | 16 September 2015 |
| | | MX 2012013467 A | 29 April 2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010382192
- WO 2011146336 A


### Non-patent literature cited in the description


- Chinese Pharmacopoeia. 2015